Europäisches Patentamt

European Patent Office

Office européen des brevets

(1) Publication number: **0 073 680**
**B1**

## EUROPEAN PATENT SPECIFICATION

(45) Date of publication of patent specification: **14.08.85**

(21) Application number: **82304583.6**

(22) Date of filing: **01.09.82**

(51) Int. Cl.⁴: **C 07 C 11/06,** C 07 C 7/148,
B 01 J 27/30

(54) Method of removal of COS from propylene.

(30) Priority: **01.09.81 US 298702**

(43) Date of publication of application:
**09.03.83 Bulletin 83/10**

(45) Publication of the grant of the patent:
**14.08.85 Bulletin 85/33**

(84) Designated Contracting States:
**BE DE FR IT NL**

(56) References cited:
FR-A-1 352 745
US-A-3 058 800
US-A-3 265 757

(73) Proprietor: **USS ENGINEERS AND
CONSULTANTS, INC.
600 Grant Street
Pittsburgh Pennsylvania 15230 (US)**

(72) Inventor: **Brownell, George, Leonard
91 Woodhaven Drive
Mount Lebanon Pennsylvania 15228 (US)**
Inventor: **Collier, Melba Jean
647 Shawnee
Houston Texas 77034 (US)**
Inventor: **Hall, William Edward
10214 Belfast Road
La Porte Texas 77571 (US)**
Inventor: **Morgan, Howard Henry
14 Valerie Circle
Monroeville Pennsylvania 15146 (US)**
Inventor: **Snyder, Arthur Raymond
4584 School Road
Export Pennsylvania 15632 (US)**

(74) Representative: **McCall, John Douglas et al
W.P. THOMPSON & CO. Coopers Building
Church Street
Liverpool L1 3AB (GB)**

Courier Press, Leamington Spa, England.

## 0 073 680

**Description**

The present invention relates to the removal of carbonyl sulfide from propylene.

Prior to the present invention, the advent of increasingly efficient and sensitive catalysts for the polymerization of propylene has caused the polypropylene industry to recognize the importance of the control of various trace impurities in the propylene feedstock. Carbonyl sulfide has been found to be one of the most troublesome impurities, causing catalyst carryover, increased ash content of product, the production of undesirable large quantities of atactic by-product, and gross inefficiencies in catalyst life conversion rate. These difficulties tend to be more pronounced and important in proportion to the increases in yield or efficiency otherwise observed in new catalysts.

COS levels in some propylene feedstocks may range from an acceptable level of less than 50 ppb (parts per billion by weight) to totally unacceptable levels of over 2 ppm (parts per million by weight). In the past, a solid NaOH bed has been used commercially, but NaOH alone is not capable commercially of lowering the COS concentration to less than 50 ppb.

Concentrations of COS in the range of a few parts per million (e.g., 1—10 ppm) are very difficult to separate from propylene by frictional distillation because the boiling point of COS differs from that of propylene by only 3.4°C. Also, COS is not completely removed from propylene by the usual sulfur-removal processes such as caustic scrubbing or amine-type scrubbing due primarily to the slow rate of hydrolysis of COS.

While it is known to hydrolyze COS over a platinum sulfide/alumina catalyst, such catalysts have not been used for the removal of small amounts of COS from propylene, possibly because of the fear of deposition of polymer on the surface of the catalyst, and the difficulty of regenerating the catalyst should such deposition occur.

We have found a catalyst of platinum sulfide on alumina may be used to hydrolyze very small amounts of COS in propylene in both the liquid and gas phases provided that certain conditions are maintained.

There is disclosed in FR—A—1 352 745 a process for removing COS from liquid propylene by hydrolysis over an alumina catalyst. However, the alumina catalyst is an alkali alumina catalyst containing 0.5 to 3% sodium or potassium. This catalyst is stated to be capable of reducing COS levels from hundreds of parts per million to tens of parts per million. By way of contrast, the present invention uses a catalyst of platinum sulfide on alumina and is capable of reducing COS levels to less than 15 parts per billion, that is, about 1000 times lower than the alkali alumina catalyst disclosed in the French Patent.

US—A—3 058 800 is very similar to the French Patent. Exactly the same catalyst is used with the same degree of COS removal, but the propylene or other hydrocarbon treated is gaseous not liquid.

According to the present invention, there is provided a method of removing carbonyl sulfide from propylene comprising passing the propylene in liquid form over a catalyst of platinum sulfide on alumina in the presence of water to hydrolyze the COS to $H_2S$ and $CO_2$.

The invention also provides a method of removing carbonyl sulfide from propylene comprising passing the propylene in gaseous form over a catalyst of platinum sulfide on alumina in the presence of water to hydrolyze the COS to $H_2S$ and $CO_2$, and regenerating the catalyst by contracting the catalyst with a solvent for polypropylene. The solvent is preferably a low boiling hydrocarbon solvent, preferably liquid propylene.

It is possible to treat propylene containing as much as 500 or more parts per million of COS to make it acceptable for use in a highly efficient ("high yield") polymerization process, that is, to reduce the COS content to below 50 parts per billion, by passing it through the catalyst bed of platinum sulfide on alumina. For such treatment to be practical and successful, there should be present in the propylene a small amount of moisture, preferably an amount of water at least double the stoichiometric amount of the COS to be hydrolyzed. The pressure may be maintained from atmospheric to 46 atmospheres (46.61 bars), preferably 13.6 to 31.6 atmospheres (13.78 to 32.02 bars) for the liquid phase or, for the vapour phase, from atmospheric to a practical limit of 82 atmospheres (83.09 bars) with about 21.4 atmospheres (21.68 bars) or 20.4 atmospheres (20.67 bars) being preferred. The temperature for the vapour phase should be 121 to 260°C at the inlet to the catalyst vessel, although for the liquid phase the temperature can be significantly lower, such as 35°C to 65°C. The flow rate of the gas will be, preferably, 1000 to 4000 $m^3$ (at atmospheric pressure and 21°C) of $C_3H_6$ vapour, per $m^3$ of catalyst per hour or, for liquid, preferably below 8 $m^3$ per hour.

Test results obtained with PtS catalyst are summarized in Table I. The feedstock consisted of liquid propylene containing 8.5 ppm COS and 10 ppm water. The catalyst bed volume was 65.6 $cm^3$ and the pressure at the inlet to the catalyst bed was 31.6 atmospheres (32.02 bars). The catalyst contained about 0.08% platinum sulfide on an alumina support. It performed well on liquid propylene at flow rates of 4 to 5 $m^3$ per hour per $m^3$ of catalyst. Above 6 $m^3$ per hour COS "breakthrough" occurred. The gas chromatograph analysis for COS was not sensitive below 15 ppb COS.

2

TABLE I

| Day | Bed Temperature °C | Flow Rate Bed volumes per hour | COS out ppb |
|-----|-----|-----|-----|
| 1 | 27 | 0.41 | <15 |
| 2 | 23 | 0.35 | <15 |
| 3 | 22 | 4.3 | 708 |
| 4 | 60 | 1.5 | <15 |
| 5 | 60 | 0.54 | <15 |
| 6 | 60 | 2.2 | <15 |
| 7 | 60 | 2.7 | <15 |
| 8 | 60 | 2.9 | <15 |
| 9 | 60 | 2.8 | <15 |
| 10 | 60 | 3.6 | <15 |
| 11 | 60 | 4.7 | <15 |
| 12 | 60 | 2.4 | <15 |
| 13 | 60 | 4.2 | <15 |
| 14 | 60 | 4.6 | <15 |
| 15 | 52 | 2.0 | <15 |
| 16 | 54 | 5.0 | <15 |
| 17 | 45 | 4.5 | <15 |
| 18 | 41 | 5.5 | <15 |
| 19 | 31 | 4.6 | 460 |

Table II represents the results of an experiment in which the temperature was maintained relatively constant at 38°C over a period of time at a pressure of 21.4 atmospheres (21.68 bars). The catalyst was the same as used for Table I. The feedstock consisted of liquid propylene containing 10 to 20 ppm COS and 40 ppm water.

TABLE II

| Time On Stream, Hrs. | Bed Volumes per Hr. | COS ppm,wt. | | $H_2S$ ppm,wt. | |
|---|---|---|---|---|---|
| | | In | Out | In | Out |
| 48 | 2.0 | 10.0 | 0.00 | 0.0 | 0.0 |
| 64 | 2.0 | 9.0 | 0.00 | 0.0 | 0.47 |
| 72 | 6.0 | 7.58 | 0.00 | 0.0 | 1.18 |
| 72.5 | 8.3 | 7.58 | 0.00 | 0.0 | 3.6 |
| 80 | 9.7 | 15.5 | 0.05 | 0.0 | 1.8 |
| 96 | 8.0 | 12.0 | 0.67 | 0.0 | 4.0 |
| 104 | 4.0 | 11.6 | 0.00 | 0.0 | 3.8 |

A commercial-size run was conducted in the vapour phase and successfully reduced the COS in a commercial propylene to acceptable levels.

In the system used to conduct the commercial-size run, the propylene containing the COS is passed first to a "$C_3$ splitter" the function of which is to remove propane from the propylene. The propylene is then passed through a heater where it is vaporized. If there is not enough water in the propylene, it may be injected upstream of the catalyst bed, typically in the form of steam. Then the propylene goes through the catalyst bed which converts COS to $CO_2$ and $H_2S$. The gas mixture is then conducted to a topping still, the function of which is to bleed off the acid gases, usually carried in a small amount of propylene. This propylene need not be lost but can be recovered by recycling to a "cracked gas" compressor or other recycling apparatus. The acid gases may be removed together with any excess water conveniently in a conventional scrubber or other treatment station such as an appropriate absorbent bed (e.g. ZnO). The "$C_3$ splitter" may be a conventional multi-stage distillation column designed to separate propylene/propane mixtures to produce "chemical grade" or "polymer grade" propylene as overhead products. The topping still is or may be a conventional multi-stage distillation column for the removal of acid gases from the product propylene. The catalyst bed is a simple cylindrical vessel with an inlet at the top and an outlet at the bottom.

In this commercial-size run, the data for COS is in terms of parts per million by volume in and parts per billion by volume out. (See Table III). The average amounts were 2.42 ppm in and 27 ppb out, or a 98.9% removal.

4

# 0 073 680

## TABLE III

| Pressure atmospheres (bars) | Temperature °C | (kg/hr) Flow Rate | Moisture ppm | COS Feed ppm | COS Effluent ppb |
|---|---|---|---|---|---|
| 20.4 (20.67) | 144 | 10,600 | 82 | 4.06 | 10 |
| 20.4 (20.67) | 147 | 8,400 | 8.9 | 2.91 | 10 |
| 20.4 (20.67) | 149 | 7,100 | 3.8 | 4.47 | 25 |
| 20.4 (20.67) | 156 | 4,600 | 7.0 | 2.40 | 70 |
| 20.1 (20.37) | 138 | 6,800 | 7.7 | 1.17 | 40 |
| 19.7 (19.96) | 143 | 7,300 | 7.3 | 2.47 | 20 |
| 20.4 (20.67) | 143 | 6,000 | 6.0 | 1.46 | 30 |
| 20.1 (20.37) | 143 | 7,500 | 7.0 | 2.18 | 30 |
| 20.1 (20.37) | 143 | 7,300 | 8.0 | 2.18 | 30 |
| 20.1 (20.37) | 143 | 5,700 | 7.0 | 2.22 | 20 |
| 20.4 (20.67) | 142 | 6,300 | 7.5 | 2.56 | 20 |

In an experimental test in a commercial-size facility to demonstrate regeneration of the catalyst, a catalyst having 0.08 weight percent platinum sulfide on an alumina support was used to remove COS from propylene in the vapour phase. The activity of the catalyst deteriorated as indicated by the fact that an increasingly higher temperature was required for COS removal and the fact that an increasing amount of COS was left in the propylene after passage of the gaseous propylene through the catalyst bed. To regenerate the catalyst, liquid propylene at 43°C and 24 atmospheres (24.32 bars) was passed through the bed to wash the catalyst surface (6 bed volumes per hour, for 4 hours). When the washed bed was again put on-line, the efficiency of COS hydrolysis improved substantially, that is, a lower bed operating temperature sufficed to reduce the COS concentration to 30 ppb. Table IV represents data for used catalyst before and after regeneration.

## TABLE IV

| Before Washing COS Concentration by Wt. | | After Washing COS Concentration by Wt. | |
|---|---|---|---|
| IN | OUT | IN | OUT |
| 3 ppm | 100 ppb | 3 ppm | 10 ppb |
| Bed Temperature to Achieve Indicated Removal — 191°C | | Bed Temperature to Achieve Indicated Removal — 124°C | |

**Claims**

1. A method of removing carbonyl sulfide from propylene by passing the propylene in liquid form over a catalyst characterised in that the catalyst is platinum sulfide on alumina and in that the propylene is passed over the catalyst in the presence of water to hydrolyze the COS to $H_2S$ and $CO_2$.

2. A method as claimed in claim 1, characterised by being conducted at 35°C to 65°C at a pressure of 13.6 to 31.6 atmospheres (13.78 to 32.02 bars).

3. A method as claimed in claim 1 or 2, characterised in that the water is present in an amount at least double the stoichiometric amount of carbonyl sulfide to be removed.

4. A method of removing carbonyl sulfide from propylene by passing the propylene in gaseous form over a catalyst characterised in that the catalyst is platinum sulfide on alumina and in that the propylene is passed over the catalyst in the presence of water to hydrolyze the COS to $H_2S$ and $CO_2$, and the catalyst is regenerated by contacting the catalyst with a solvent for polypropylene.

5

5. A method as claimed in claim 4, characterised in that the solvent is a low boiling hydrocarbon solvent.

6. A method as claimed in claim 5, characterised in that the solvent is liquid propylene.

7. A method as claimed in any one of claims 4 to 6, characterised in that the hydrolysis is conducted at 121°C to 260°C at a pressure above atmospheric.

8. A method as claimed in any one of claims 4 to 7, characterised in that the water is present in an amount at least double the stoichiometric amount of carbonyl sulfide to be removed.

**Revendications**

1. Procédé d'élimination de sulfure de carbonyl à partir de propylène par passage du propylène sous forme liquide sur un catalyseur, caractérisé en ce que le catalyseur est du sulfure de platine sur de l'alumine et que le propylène est envoyé sur le catalyseur en présence d'eau pour hydrolyser le SCO en $H_2S$ et $CO_2$.

2. Procédé suivant la revendication 1, caractérisé en ce qu'il est conduit entre 35° et 65°C, sous une pression de 13,6 à 31,6 atmosphères (13,78 à 32,02 bars).

3. Procédé suivant la revendication 1 ou 2, caractérisé en ce que l'eau est présente en une quantité au moins double de la quantité stoechiométrique de sulfure de carbonyle à éliminer.

4. Procédé d'élimination de sulfure de carbonyle à partir de propylène sous forme gazeuse sur un catalyseur, caractérisé en ce que le catalyseur est du sulfure de platine sur de l'alumine; que le propylène est envoyé sur le catalyseur en présence d'eau pour hydrolyser le SCO en $H_2S$ et $CO_2$ et que le catalyseur est régénéré par contact de celui-ci avec un solvant pour le polypropylène.

5. Procédé suivant la revendication 4, caractérisé en ce que le solvant est un solvant hydrocarboné à bas point d'ébullition.

6. Procédé suivant la revendication 5, caractérisé en ce que le solvant est du propylène liquide.

7. Procédé suivant l'une quelconque des revendications 4 à 6, caractérisé en ce que l'hydrolyse est conduite entre 121 et 260°C sous une pression supérieure à la pression atmosphérique.

8. Procédé suivant l'une quelconque des revendications 4 à 7, caractérisé en ce que l'eau est présente en une quantité au moins double de la quantité stoechiométrique de sulfure de carbonyle à éliminer.

**Patentansprüche**

1. Verfahren zur Entfernung von Carbonylsulfid aus Propylen, indem das Propylen in flüssiger Form über einen Katalysator geleitet wird, dadurch gekennzeichnet, daß der Katalysator Platinsulfid auf Tonerde ist und daß das Propylen in Gegenwart von Wasser über den Katalysator geleitet wird, um das COS zu $H_2S$ und $CO_2$ zu hydrolysieren.

2. Verfahren nach Anspruch 1, dadurch gekennzeichnet, daß es bei 35 bis 65°C und unter einem Druck von 13,6 bis 31,6 atm (13,78 bis 32,02 bar) durchgeführt wird.

3. Verfahren nach Anspruch 1 oder 2, dadurch gekennzeichnet, daß das Wasser mindestens in der doppelten Menge der stöchiometrischen Menge des zu entfernenden Carbonylsulfids vorhanden ist.

4. Verfahren zur Entfernung von Carbonylsulfid von Propylen, indem gasförmiges Propylen über einen Katalysator geleitet wird, dadurch gekennzeichnet, daß der Katalysator Platinsulfid auf Tonerde ist und daß das Propylen in Gegenwart von Wasser über den Katalysator geleitet wird, um das COS zu $H_2S$ und $CO_2$ zu hydrolysieren, und der Katalysator regeneriert wird, indem man ihn mit einem Lösungsmittel für Propylen in Berührung bringt.

5. Verfahren nach Anspruch 4, dadurch gekennzeichnet, daß das Lösungsmittel ein leichtsiedendes Kohlenwasserstoff-Lösungsmittel ist.

6. Verfahren nach Anspruch 4, dadurch gekennzeichnet, daß das Lösungsmittel flüssiges Propylen ist.

7. Verfahren nach Anspruch 4 bis 6, dadurch gekennzeichnet, daß die Hydrolyse bei 121 bis 260°C und unter einem Druck, der höher als der Luftdruck ist, durchgeführt wird.

8. Verfahren nach Anspruch 4 bis 7, dadurch gekennzeichnet, daß das Wasser mindestens in der doppelten Menge der stöchiometrischen Menge des zu entfernenden Carbonylsulfids vorhanden ist.